# EUROPEAN PATENT SPECIFICATION

(11) **EP 0 687 658 B1**
(45) Date of publication and mention of the grant of the patent: **21.01.1998**
(21) Application number: 95108171.0
(22) Date of filing: 29.05.1995
(51) Int. Cl.: C07C 2/54, B01J 31/40

(54) **Process for the recovery of a strong acid from an aqueous solution**
Verfahren zur Rückgewinnung eines starken Säures aus einer wässrigen Lösung
Procédé pour la récupération d'acide fort à partir d'une solution aqueuse

(30) Priority: 13.06.1994 DK 67394; 17.06.1994 DK 70794
(43) Date of publication of application: 20.12.1995
(73) Proprietor: Haldor Topsoe A/S, DK-2800 Lyngby (DK)
(72) Inventor: Hommeltoft, Sven Ivar, DK-3400 Hillerod (DK)
(74) Representative: Grünecker, Kinkeldey, Stockmair & Schwanhäusser Anwaltssozietät

(56) References cited:
- US-A- 5 220 095

## Description

The present invention is related to the recovery of strong acids from an aqueous solution, and, in particular, to a process, wherein the acid is regained in its anhydrous form from water by a distillation process.

Strong acids are employed in the chemical industry as useful catalysts in a large number of hydrocarbon conversion processes. As an example, hydrogen fluoride and sulphuric acids are used in large quantities in the alkylation of hydrocarbons to produce high quality gasoline. Use of perfluorinated sulphonic acids, as catalyst in the alkylation process, has recently been described in the literature.

In order to meet environmental requirements and to improve process economy in a process being catalyzed by a strong acid, it is desirable to recover and to reuse the acid from a process effluent stream containing recoverable quantities of spent acid.

Simple recovery methods would comprise extraction of spent acid into an aqueous phase and subsequent removal of water by distillation or evaporation.

Because of strong interactions between water and the acid, distillation of diluted aqueous solution of the acid, however, results in recovery of the acid in its hydrate form. Acid hydrates are catalytic inactive, and it is, therefore, necessary further to process recovered acid hydrate to its anhydrous form.

It has now been found that addition of a week base to an aqueous solution of a strong acid provides recovery of the acid in its anhydrous form by a sequence of evaporation of water and subsequent recovery of the anhydrous acid by distillation.

As a theoretical explanation, the weak base interacts with acid protons and competes, thereby, with water molecules leaving water more volatile than it would be solely in combination with the acid. After removal of water, it is possible to distil off the acid from the mixed base acid phase in its anhydrous form.

Pursuant to this finding, the present invention provides a process for the recovery of an acid in its anhydrous form from an aqueous solution containing the acid in its hydrated form, comprising the steps of adding to the solution a weak base in form of a soluble salt of the acid;
evaporating water from the solution containing the acid hydrate and the salt of the acid to a substantial water free acid salt mixture, and finally
distilling off the acid in its anhydrous form from the mixture.

Based on the above theoretical explanation, proper bases for use in the process are selected from salts, which interact with the acid hydrate.

Suitable bases include alkyl ammonium, pyridine, pyrrolidine and/or alkyl substituted pyridine and pyrrolidine, and cesium salts of the acid to be recovered, such as salts with trialkyl amine.

The invention is, as mentioned before, useful in the recovery of acids being catalytic active in the alkylation of a paraffinic hydrocarbon feedstock. Those processes conventionally employ hydrogen fluoride as alkylation catalyst. Alkylation process being catalyzed by a perfluorinated sulphonic acid is further known from U.S. Patent No. 5,220,095. In the known alkylation processes, considerable economic and environmental advantages are gained when recovering spent acid catalyst by simple extraction from an alkylation effluent stream with water and processing the aqueous extract in accordance with the above recovery process.

Accordingly, the invention further provides improvements in the alkylation of a hydrocarbon feedstock in presence of an acid catalyst comprising the steps of extracting with water an alkylation effluent stream containing spent acid catalyst;
adding to the aqueous extract a salt of the acid catalyst;
concentrating the salt containing extract;
distilling off the acid from the concentrated extract; and
recycling the acid to the alkylation process.

The steps of concentrating and distillating off the acid during the acid recovery process may be carried out conventionally by removing excess of water from the acid hydrate salt mixture at reduced pressure in an evaporation column and then cycling the mixture to a distillation column for recovery of the anhydrous acid. Remaining amounts of the acid salt mixture may then be recycled from the distillation column to the evaporation column.

Alternatively, the acid may be recovered in its anhydrous form in a continuous or flash distillation process by passing the solution of the acid salt mixture over a hot surface at a temperature or pressure, where water continuously evaporates from the mixture. In a subsequent step, the dehydrated mixture is passed over a further hot surface at higher temperature and/or lower pressure and the acid is continuously distilled off while the base is recycled to the first step.

In the following Examples specific embodiments of the invention are described in details.

### Example 1

150 g (1.00 mole) trifluoromethane sulphonic acid (CF₃SO₃H) were poured on ice to form an aqueous solution. To the solution 55.5 g (0.55 mole) triethyl amine were added and excess water evaporated at a temperature of 90°C, and a pressure of about 50 mbar. The evaporated solution contained 230 g of a salt/acid mixture with a content of water of 11.2%.

220 g of this mixture (equivalent to 0.53 mole triethyl ammonium triflate + 0.43 mole CF₃SO₃H + 1.37 mole water) were distilled from an oil bath at 1.5-2.0 mbar. The mixture started to boil at a bath temperature of 65°C. The distillate could not be condensed at room temperature, but was collected in a freezing trap at -196°C. 24.4 g water were collected in the freezing trap. At a bath temperature of 190-220°C a distillate, which initially condensed at 95°C, was collected. As a first fraction 7.68 g distillate consisting of CF₃SO₃H containing 4% water (0.05 mole acid) were collected at a condensation temperature of 95-90°C. The condensation temperature felt gradually to 44°C and a second fraction of distillate consisting of 32.2 g substantially anhydrous acid containing 0.36% water (0.21 mole) CF₃SO₃H was obtained. The remanence of the distillation consisted of 154.2 g salt/acid mixture with less than 0.1% water.

### Example 2

21.2 g (0.10 mole) trimethyl ammonium triflate were mixed with 25.7 g (0.15 mole) CF₃SO₃H · H₂O (trifluoromethanesulphonic acid monohydrate) and distilled at 2 mbar from an oil bath. A pre-run of water (1.63 g, 0.09 mole) and two distillate fractions were collected as described in Example 1. The first fraction contained 15.84 g acid with a water content of 6.4% (0.056 mole water + 0.10 mole trifluoromethanesulphonic acid). The second fraction consisted of 5.1 g acid containing less than 0.2% water (0.033 mole trifluoromethanesulphonic acid).

### Example 3

19.87 g (79 mmole) triethylammonium triflate were mixed with 10.34 g (62 mmole) trifluoromethanesulphonic acid hydrate and distilled at a pressure of 2 mbar from an oil bath. As the oil bath reached 90°C, the mixture boiled and the distillate did not condense at room temperature. The distillate was trapped in a freezing trap at -196°C. 1.03 g (57 mmole) water were found in the freezing trap.

At a bath temperature of 210-222°C 3.72 g distillate condensing at 42-46°C were collected. The distillate contained 3.72 g trifluoromethanesulphonic acid (24 mmole) with <0.1% water. Remaining in the distillation flask were 24.72 g acid/salt mixture containing 66.8% (111 mmole) triethylammonium triflate.

### Example 4

Distillation from tetraethylammonium triflate. To a flask containing 74.64 g (0.10 mole) 20% tetraethylammonium hydroxide (Et₄N⁺OH⁻) cooled in ice bath were added 37.62 g (0.25 mole) trifluoromethanesulphonic acid, resulting in an aqueous solution of 0.1 mole tetraethylammonium triflate and 0.15 mole of trifluoromethanesulphonic acid.

Most of the water was evaporated leaving 60.95 g of a mixture containing 15.9% (w/w) water.

57.05 g of the mixture were distilled as described above yielding 8.9 g of an aqueous pre-run containing 0.14 g trifluoromethanesulphonic acid and two distillate fractions. The first fraction consisted of 2.50 g trifluoromethanesulphonic acid with a water content of 3.3% and the second fraction consisted of 9.01 g trifluoromethanesulphonic acid with 0.84% water content.

### Example 5

Distillation of trifluoromethanesulphonic acid from methyl-pyrrolidine salt. A mixture of 44.53 g (0.297 mole) trifluoromethanesulphonic acid, 9.65 g (0.536 mole) water and 8.43 g (0.103 mole) methyl pyrrolidine was distilled as described above. 7.6 g of an aqueous pre-run were obtained, which was collected in the freezing trap. 15.4 g of a first fraction containing trifluoromethanesulphonic acid with a water content 12.4% (w/w) corresponding to 13.5 g (0.09 mole) trifluoromethanesulphonic acid and a second fraction consisting of 10.0 g trifluoromethanesulphonic acid with a water content of 0.80% (w/w) (corresponding to 0.066 mole trifluoromethanesulphonic acid) were collected.

### Example 6

Distillation of pentafluorethanesulphonic acid from its triethylamine salt.

To 3.77 g (0.21 mole) water in 39.86 g (0.199 mole) C₂F₅SO₃H were added 10.64 g (0.105 mole) triethyl amine resulting in a mixture of 0.105 mole Et₃NH⁺C₂F₅SO₃⁻, 0.094 mole C₂F₅SO₃H and 0.21 mole water. This mixture was distilled as described above to 3.28 g aqueous pre-run (in freezing trap) and 13.47 g acid with a water content of 0.4%, corresponding to 0.067 mole C₂F₅SO₃H.

### Example 7

Distillation of trifluoromethanesulphonic acid from its cesium salt.

29.65 g trifluoromethanesulphonic acid were added to 33.05 g 50 %(w/w) CsOH (0.110 mole CsOH, 0.92 mole water) forming a partially crystallized mixture. The mixture was distilled under vacuum as described above and a fraction containing 5.84 g of acid with a water content of 6.4% (w/w) was collected.

### Example 8

Distillation of trifluormethanesulphonic acid from its pyridine salt.

To 4.4 g (0.24 mole) water in 30.33 g (0.20 mole) CF₃SO₃H were added 9.6 g (0.121 mole) pyridine. The obtained mixture was distilled as described above and 6.76 g acid with a water content of 0.9% (corresponding to 0.045 mole trifluoromethanesulphonic acid) were recovered.

### Example 9

Recovery of trifluormethanesulphonic acid from acid soluble oil formed as a by-product in an isobutane alkylation process.

The trifluoromethanesulphonic acid in an ASO/acid mixture (approx. 60% (w/w) trifluoromethanesulphonic acid) formed as by-product in a trifluoromethanesulphonic acid catalyzed alkylation process, was extracted with water to yield an aqueous solution containing 24.4% (w/w) trifluoromethanesulphonic acid.

55.05 g of the aqueous trifluoromethanesulphonic acid solution (13.4 g, 0.090 mole) obtained by extraction of the acid soluble oil mixture withdrawn from an alkylation reactor were added to the pyridine salt remanence left from Example 8 (7.4% (w/w) or 2.2 g (0.015 moles) trifluoromethanesulphonic acid content). Distillation of the obtained mixture resulted in the recovery of a fraction containing 8.5 g trifluoromethanesulphonic acid with a water content of 0.9% (w/w).

### Example 10

Distillation of pentafluoroethanesulphonic acid from triethylammonium triflate.

A mixture of 18.78 g (0.094 mole) C₂F₅SO₃H, 26.20 g (0.104 mole) Et₃NH⁺CF₃SO₃⁻ and 4.09 g (0.227 mole) water was distilled as described above. 8.54 g product distillate were collected. The distillate contained 1.4% (w/w) water in a mixture of C₂F₅SO₃H and CF₃SO₃H in the weight ratio of 10:9.

### Example 11

### Flash distillation.

In this Example, water was removed from an acid base mixture by flash distillation by passage of the mixture over a hot surface at conditions, where continuous evaporation of water is provided.

The outside of the heated surface in form of a glass spiral was heated to 215°C by condensing vapour of dodecane. The pressure inside the spiral was adjusted to 25 mbar.

A feed mixture containing 62 % (w/w) triethylammonium triflate, 29 %(w/w) trifluoromethanesulphonic acid and 9 %(w/w) water were fed at a rate of 1.88 g/min. and the dehydrated acid mixture was collected in a collector at the outlet of the spiral. The mixture contained 0.21 %(w/w) water and 25% trifluoromethanesulphonic acid. The remainder of the acid was found in the aqueous distillate in a product collector connected to the inlet of the spiral.

### Example 12

### Acid recovery.

In a process, similar to the process of Example 11, the outside of the heated surface of the glasspiral was heated to 215°C by condensing vapour of dodecane and the pressure inside the spiral was adjusted to 1 mbar. A feed mixture containing 65 %(w/w) triethylammonium triflate, 35 %(w/w) trifluoromethanesulphonic acid and 0.3 %(w/w) water were fed at a rate of 1.97 g/min. and the acid was collected in the product collector at a rate of 0.5 g/min corresponding to 73% recovery.

### Example 13

Simultaneous water stripping and acid recovery. Equipment used in this Example consists of two distillation units connected in series. A first Unit was used for water stripping. The Unit was heated with condensing n-decane (174°C) from an evaporator. The Unit was kept under a pressure of 25-30 mbar. A second Unit was used as acid recovery unit and was heated by condensing n-dodecane (216°C) from an evaporator and kept at a pressure of 2.5-3 mbar.

A feed mixture with the same composition as in Example 11 was fed into the first unit at a rate of 1.8 g/min. In the first unit, the mixture passed through a glass spiral being heated at the outside by condensing n-decane. By passage through the spiral, water was stripped of the mixture and collected in a water cooled collector. The substantial water free mixture leaving the spiral was then introduced into a second glass spiral in the second Unit. When passing through the second spiral, the acid in its dehydrated form was stripped of the acid salt mixture and collected in a water cooled acid collector at a rate of 0.25 g/min. Residual acid salt mixture was withdrawn from the second Unit at the outlet of the spiral.

### Example 14

Example 14 was carried out similar to Example 13, with the exception that the second acid recovery Unit was heated by condensing tetradecane (252°C), whereas the first Unit was heated by condensing decane (174°C).

A feed mixture with the same composition as in Example 1 was fed to the first unit at a rate of 2.8 g/min., and the recovered acid collected in the acid collector of the second unit at a rate of 0.59 g/min. The water content of the recovered acid was 3.0 %(w/w).

### Example 15

### Regeneration of acid oil (ASO) from alkylation process.

Trifluoromethanesulphonic acid in an ASO/acid mixture (approx. 60% (w/w) trifluoromethanesulphonic acid) formed as by-product in a trifluoromethanesulphonic acid catalyzed alkylation process, was extracted with water to yield an aqueous solution containing 24.4% (w/w) trifluoromethanesulphonic acid.

112.2 g of this solution (27.4 g, 0.18 mole TfOH) were concentrated by boiling off 71.8 g of water at atmospheric pressure leaving the acid as 40.4 g concentrate (calculated acid content: 67.7% (w/w)). The concentrate was combined with a 71.4 g salt mixture (acid content: 6.7% (w/w)) from a previous acid recovery experiment performed as in Example 14.

In this Example the first Unit was heated by condensing undecane (196°C), and the seond Unit was heated by condensing tetradecane (252°C). The combined mixture was fed to the first Unit at a rate of 1.8 g/min., and an acid with a water content of 2.0% (w/w) was recovered at a rate of 0.4 g/min. The acid base mixture recovered contained 7.4% (w/w) trifluoromethanesulphonic acid.

## Claims

1. A process for the recovery of an acid in its anhydrous form, from an aqueous solution containing the acid in its hydrated form comprising the steps of
adding to the solution a weak base in form of a soluble salt of the acid;
concentrating the solution containing the acid hydrate and the salt of the acid to a substantial water free acid salt mixture, and finally
distilling off the acid in its anhydrous form from the acid salt mixture.

2. Process according to claim 1, wherein the week base comprises a salt of the acid with trialkyl amine.

3. Process according to claim 2, wherein the trialkyl amine is trimethyl and/or triethyl amine.

4. Process according to claim 1, wherein the week base comprises a salt of the acid with pyridine and/or alkylpyridine.

5. Process according to claim 1, wherein the week base comprises a salt of the acid with pyrrolidine and/or alkyl pyrrolidine.

6. Process according to claim 1, wherein remaining amounts of the acid salt mixture in the distillation steps are recycled to the salt addition step.

7. Process according to claim 1, wherein the concentration of the solution containing the acid hydrate and the salt of the acid to a substantial water free acid salt mixture is carried out continuously by passing the solution over a hot surface at elevated temperature and/or reduced pressure and continuously evaporating water from the solution.

8. Process according to claim 1, wherein the acid is distilled off in its anhydrous form from the acid salt mixture continuously by passing the mixture over a hot surface at elevated temperature and/or reduced pressure.

9. Use of a process according to anyone of the preceding claims for the recovery of an acid from acid soluble oil formed in the alkylation of a hydrocarbon feedstock.

## Patentansprüche

1. Verfahren zur Rückgewinnung einer Säure in ihrer wasserfreien Form aus einer wäßrigen Lösung, welche die Säure in ihrer hydratisierten Form enthält, das die Stufen umfaßt:
Zugabe einer schwachen Base in Form eines löslichen Salzes der Säure zu der Lösung;
Einengen der das Säurehydrat und das Salz der Säure enthaltenden Lösung zu einer im wesentlichen wasserfreien Säure/Salz-Mischung und schließlich Abdestillieren der Säure in ihrer wasserfreien Form aus der Säure/Salz-Mischung.

2. Verfahren nach Anspruch 1, worin die schwache Base ein Salz der Säure mit Trialkylamin umfaßt.

3. Verfahren nach Anspruch 2, worin das Trialkylamin Trimethylamin und/oder Triethylamin ist.

4. Verfahren nach Anspruch 1, worin die schwache Base ein Salz der Säure mit Pyridin und/oder Alkylpyridin umfaßt.

5. Verfahren nach Anspruch 1, worin die schwache Base ein Salz der Säure mit Pyrrolidin und/oder Alkylpyrrolidin umfaßt.

6. Verfahren nach Anspruch 1, worin die in den Destillationsstufen zurückbleibenden Mengen an Säure/Salz-Mischung in die Salz-Zugabe-Stufe recyclisiert werden.

7. Verfahren nach Anspruch 1, worin die Einengung der das Säurehydrat und das Salz der Säure enthaltenden Lösung zu einem im wesentlichen wasserfreien Säure/Salz-Gemisch kontinuierlich durchgeführt wird durch Überleiten der Lösung über eine heiße Oberfläche bei erhöhter Temperatur und/oder vermindertem Druck und kontinuierliches Verdampfen von Wasser aus der Lösung.

8. Verfahren nach Anspruch 1, worin die Säure in ihrer wasserfreien Form aus der Säure/Salz-Mischung kontinuierlich abdestilliert wird durch Überleiten der Mischung über eine heiße Oberfläche bei erhöhter Temperatur und/oder vermindertem Druck.

9. Anwendung eines Verfahrens nach einem der vorhergehenden Ansprüche zur Rückgewinnung einer Säure aus einem säurelöslichen Öl, das bei der Alkylierung eines Kohlenwasserstoff-Ausgangsmaterials gebildet wird.

## Revendications

1. Procédé pour récupérer un acide sous sa forme anhydre, à partir d'une solution aqueuse contenant l'acide sous sa forme hydratée, comprenant les étapes
d'addition à la solution d'une base faible sous forme d'un sel soluble de l'acide ;
de concentration de la solution contenant l'hydrate d'acide et le sel de l'acide en un mélange sel acide substantiellement exempt d'eau, et finalement,
de distillation de l'acide sous sa forme anhydre à partir du mélange sel acide.

2. Procédé selon la revendication 1, dans lequel la base faible comprend un sel de l'acide avec de la trialkylamine.

3. Procédé selon la revendication 2, dans lequel la trialkylamine est la triméthyl et/ou triéthylamine.

4. Procédé selon la revendication 1, dans lequel la base faible comprend un sel de l'acide avec de la pyridine et/ou de l'alkylpyridine.

5. Procédé selon la revendication 1, dans lequel la base faible comprend un sel de l'acide avec de la pyrrolidine et/ou de l'alkylpyrrolidine.

6. Procédé selon la revendication 1, dans lequel les quantités restantes du mélange sel acide dans les étapes de distillation sont recyclées à l'étape d'addition de sel.

7. Procédé selon la revendication 1, dans lequel la concentration de la solution contenant l'hydrate d'acide et le sel de l'acide en un mélange sel acide substantiellement exempt d'eau est effectuée en continu en faisant passer la solution sur une surface chaude à température élevée et/ou pression réduite et en évaporant en continu l'eau de la solution.

8. Procédé selon la revendication 1, dans lequel l'acide est distillé sous sa forme anhydre, à partir du mélange sel acide en continu en faisant passer le mélange sur une surface chaude à température élevée et/ou pression réduite.

9. Utilisation d'un procédé selon l'une quelconque des revendications précédentes, pour récupérer un acide à partir d'une huile soluble dans un acide formée lors de l'alkylation d'une charge d'alimentation hydrocarbonée.
